# EUROPEAN PATENT SPECIFICATION

(11) **EP 2 790 648 B1**
(45) Date of publication and mention of the grant of the patent: **05.04.2017**
(21) Application number: 11808064.7
(22) Date of filing: 16.12.2011
(51) Int. Cl.: A61Q 11/00, A61K 8/24, A61K 8/28, A61K 8/42, A61K 8/58, A61K 8/73, A61K 8/02, A61K 8/19

(54) **COLOR CHANGING ORAL COMPOSITIONS CONTAINING FILM**
FARBÄNDERNDE ORALE ZUSAMMENSETZUNGEN ENTHALTEND EINEN FILM
COMPOSITIONS ORALES CAPABLES DE CHANGER DE COULEUR CONTENANT DU FILM

(43) Date of publication of application: 22.10.2014
(73) Proprietor: Colgate-Palmolive Company, New York, NY 10022 (US)
(72) Inventor: PAN, Guisheng, Philadelphia, Pennsylvania 19114 (US); SZEWCZYK, Gregory, Flemington, New Jersey 08822 (US); LIN, Nora, Basking Ridge, New Jersey 07920 (US)
(74) Representative: Jenkins, Peter David
(86) International application number: PCT/US2011/065311
(87) International publication number: WO 2013/089762

(56) References cited:
- WO-A1-2006/119286
- WO-A1-2010/114551
- US-A1- 2005 136 096
- US-A1- 2011 150 968
- DATABASE CA [Online] CHEMICAL ABSTRACTS SERVICE, COLUMBUS, OHIO, US; 1985, "Toothpastes containing color indicators for timing adequate brushing", XP002683277, Database accession no. 102:172447 & JP 60 016913 A (LION CORP) 28 January 1985 (1985-01-28)
- DATABASE CA [Online] CHEMICAL ABSTRACTS SERVICE, COLUMBUS, OHIO, US; 1985, "Toothpastes containing color indicators for timing adequate brushing", XP002683278, Database accession no. 102:172448 & JP 60 016912 A (LION CORP) 28 January 1985 (1985-01-28)
- ANONYMOUS: "SODIUM ALGINATE (ALGINATE, ALGIN)", MOLECULAR RECIPES, 12 June 2015 (2015-06-12), XP055195456, Retrieved from the Internet: URL:http://www.molecularrecipes.com/hydroc olloid-guide/sodium-alginate-alginate-algi n/ [retrieved on 2015-06-12]
- DATABASE WPI Derwent Publications Ltd., London, GB; AN 1985-059702 & JP S6 016913 A 28 January 1985

## Description

### BACKGROUND

It is recommended that children should brush their teeth for at least 45-60 seconds, and adults for at least 90 to 120 seconds. Most people, especially children, do not brush their teeth for a sufficient period of time to obtain maximum benefit, and moreover have difficulty accurately estimating the time necessary to brush the teeth.

There is a need for improved, consumer-friendly products and methods to encourage children to brush their teeth for a longer period of time.

WO 2010/114551 discloses personal care products containing multilayer films. JP60016913 discloses compositions for oral cavity application comprising particles composed of a pigment and water-soluble polymer, where the particles are coated with inorganic powder. JP60016912 discloses particles composed of a pigment and a water-insoluble or hardly soluble polymer covered with inorganic powder.

### SUMMARY

In a first aspect, the present invention provides an orally acceptable powder-coated dissolvable single-layer film; wherein the film comprises a polymer matrix and wherein the matrix comprises a pigment that is released upon dissolution of the matrix; wherein the powder coating comprises a pigment, and wherein the powder coating further comprises a water-insoluble antimicrobial agent.

In a second aspect, the present invention provides a method of making a film as defined above, comprising forming a dissolvable polymer matrix, distributing a powder on the surface of the matrix, and heating the powder-coated film until the powder is adsorbed to the matrix.

Further areas of applicability of the present invention will become apparent from the detailed description provided hereinafter.

### DETAILED DESCRIPTION

The following description of the preferred embodiment(s) is merely exemplary in nature and is in no way intended to limit the invention, its application, or uses.

The invention thus provides, in a first embodiment, an orally acceptable powder-coated dissolvable single-layer film, wherein the film comprises a polymer matrix and wherein the matrix comprises a pigment that is released upon dissolution of the matrix, wherein the powder coating comprises a pigment, and wherein the powder coating further comprises a water-insoluble antimicrobial agent (Film 1), for example:
1.1.
1.2. Film 1 wherein the film matrix comprises cellulose ethers, e.g., selected from
   (i) alkylcellulose, e.g., methylcellulose;
   (ii) hydroxyalkyl cellulose, e.g., selected from hydroxypropyl methyl cellulose, hydroxyethylpropyl cellulose, hydroxybutyl methyl cellulose, hydroxy propyl methyl cellulose, carboxymethyl cellulose and mixtures thereof;
   and (iii) mixtures thereof;
1.3. Any of the foregoing films wherein the film matrix comprises a starch, e.g. a pregelatinized starch;
1.4. Any of the foregoing films wherein the film matrix comprises a plasticizer, e.g, a polyalcohol, e.g., sorbitol, propylene glycol, glycerol, or low molecular weight polyethylene glycol, e.g., PEG 200;
1.5. Any of the foregoing films wherein the film matrix comprises propylene glycol, e.g., in an amount effective to provide plasticity to the film, e.g., about 20-30% by dry weight of the film;
1.6. Any of the foregoing films wherein the film matrix comprises a non-ionic surfactant or emulsifier, e.g., a polysorbate, e.g., polysorbate 80 (also known as polyoxyethylene(20) sorbitan monooleate, available commercially e.g., as Tween® 80), e.g., in an amount of about 1-5% by dry weight of the film;
1.7. Any of the foregoing films wherein the film matrix comprises a pigment or combination of pigments, e.g., selected from a red pigment, for example D&C Red 30, a green pigment, for example Pigment Green 7, a yellow pigment, e.g. (Natpure LC 128 Yellow, from Sensient Co.), a blue pigment, for example a phthalocyanine, for example Pigment Blue 15: 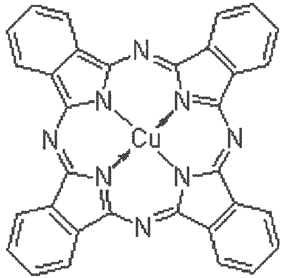
1.8.
1.9. Any of the foregoing films wherein the powder coating comprises a white powder pigment, e.g., comprising particles of titanium dioxide or Ca₂P₂O₇;
1.10. Any of the foregoing films wherein the powder coating comprises a red powder pigment, e.g., comprising particles of red iron oxide or D&C Red 30;
1.11. Any of the foregoing films wherein the film matrix comprises a polar pigment;
1.12. Any of the foregoing films wherein the powder coating comprises a polar compound;
1.13. Any of the foregoing films wherein the powder coating comprises a water-insoluble material;
1.14. Any of the foregoing films wherein the powder coating comprises a water-insoluble antimicrobial agent selected from triclosan and zinc oxide;
1.15. Any of the foregoing films wherein the film matrix is substantially dissolved after a period of greater than 30 seconds and less than 180 seconds of brushing in the presence of water;
1.16. Any of the foregoing films wherein the average thickness of the film is 1-3 mil;
1.17. Any of the foregoing films which is made by a process of forming a dissolvable film matrix, distributing a powder on the surface of the film matrix, and heating the powder-coated film until the powder is adsorbed to the film;
1.18. Any of the foregoing films wherein the film matrix comprises, by dry weight of the matrix, (i) 20-60% cellulose ethers selected from methyl cellulose, hydroxypropylmethyl cellulose, and mixtures thereof, (ii) 10-30% propylene glycol; 1-5% polysorbate 80, and 15-55% pigment.

The invention further provides an oral care product, e.g., a dentifrice, for example a toothpaste, e.g., a clear gel toothpaste, comprising an orally acceptable powder-coated dissolvable film according to any of Film 1, et seq., e.g.
a. wherein the powder-coated dissolvable film comprises a pigment in the film matrix, e.g.,
b. wherein upon use, the film matrix dissolves following at least 30 seconds and not more than about 180 seconds, e.g., about 45-60 seconds in a toothpaste for use by a child and about 90-120 seconds in a toothpaste for use by an adult, thereby releasing the pigment and providing a color signal to the user of adequate brushing.
For example, in one embodiment, the toothpaste is a clear gel, in which the film particles can be seen clearly. The film particles may be, for example, small squares 2-4 mm across. They may be all one color or assorted colors, the color being imparted by the powder coating, and the film matrix may contain a high concentration of pigment. After a period of brushing, e.g., at least 30 seconds, the film matrix is disrupted, and the clear gel toothpaste is suddenly colored by the pigment, signaling to the user that he or she has brushed for an adequate period.

The description further provides a method of cleaning the teeth comprising brushing with a toothpaste comprising an orally acceptable powder-coated dissolvable film, e.g, as described in the preceding paragraph, for example
a. the method wherein the powder-coated dissolvable film comprises a pigment in the film matrix and brushing is continued until the film matrix dissolves and the pigment provides a color signal to the user of adequate brushing, for example,
b. the foregoing method when the brushing time before the film matrix dissolves is between 30 and 180 seconds, e.g., about 45-60 seconds for a toothpaste for use by a child and about 90-120 seconds for a toothpaste for use by an adult.

The invention further provides a method of manufacturing a powder-coated dissolvable film according to any of Film 1, et seq. comprising forming a dissolvable film matrix, distributing a powder on the surface of the film matrix, and heating the powder-coated film until the powder is adsorbed to the film.

*Orally acceptable:* The compositions of the invention are intended for topical use in the mouth, thus components for use in the present invention should be orally acceptable, that is, safe for topical use in the mouth, in the amounts and concentrations provided.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight.

### EXAMPLE

### Reference Example 1

Blue 15 pigment is encapsulated in films to trigger color change during brushing. Single layer films are less expensive to make but not attractive to some consumers, as the high pigment load makes them appear black. Triple layer white-black-white films are made to hide the black films to make white films, which are more attractive, but these triple films are very expensive. The goal is to transform the black films into other colors by simple, less expensive powder coating method compared to the making of triple layer films.

A prototype color change toothpaste product is developed by encapsulating the pigment into a dissolvable polymer film. During brushing, the films swell from water and disintegrate, releasing the pigment and, thus, color change occurs to indicate the consumer when the brushing is done. The goal is to use color change signal to increase the brushing time for the consumer by controlling the brushing time at 45-60 sec for children and 90-120 sec for the adults.

Two film options are developed: (1) single layer black films which are less expensive and have very strong color change signal (high color change contrast), (2) triple layer white-black-white films which hide the black film in the middle. These triple layer films are more consumer-acceptable visually, however the color change contrast is diminished due to the very high color-covering power of TiO2 from the outer layers, and are very expensive to make.

To balance the advantages and disavantages for the above options, we adsorb other powders/colors onto the black film surface to convert the black films into other color films. This method avoids the cost and complexity of creating additional film layers appended to the core film.

A prototype film is made making a slurry in water using the following ingredients then drying to obtain a film having an average thickness of about 1 mil:

| **Ingredient** | Weight % of slurry | Weight % of dry film |
|---|---|---|
| Water | 66.8 | 0 |
| Hydroxypropylmethyl cellulose (Methocel E5) | 7 | 21.1 |
| Pigment (Blue 15) | 17 | 51.2 |
| Propylene Glycol | 8 | 24.1 |
| Polysorbate (Tween 80) | 1.2 | 3.6 |
| Total Amount | 100 | 100 |

The film appears black, due to the high pigment concentration. The pigment, Pigment Blue 15, has a large π-conjugated system and is highly polar. It thus has a very high polarizability that renders the film very sticky. The film is difficult to remove from glass, metal, and plastic surfaces because the thin film is very sticky and cannot be peeled off. Low energy surface TEFLON® sheet and releasing agents are therefore used to cast the films in order to permit separation of the film from the surface easily. This sticky black film readily retains wet powder on its surface.

*White powder coated films:* Ca₂P₂O₇ and a small amount of water are used to coat small square film flakes. The small amount of water is used as a glue for the films because the films are soluble in water; too much water will dissolve the film, but a little water will make the films sticky. The wet Ca₂P₂O₇ powder is mixed with the film flakes. It binds to the black film, which creates a coating that turns the film white. Alternatively, TiO₂ is used as the white pigment. The resulting white film is then heated to remove any residual water, and mixed into a clear gel toothpaste. The film fragments are stable in the gel toothpaste until use. Upon use, the clear gel toothpaste turns blue after about 45 seconds of brushing, when the film fragments disintegrate and release the blue pigment.

Alternatively, 20% glycerin+80% ethanol is used to replace water as the "glue" since water may dissolve the films but the glycerin and ethanol will not. Glycerin is used as a soluble adhesive. It binds the powder pigment to the films better than pure ethanol first, and then dissolve away when mixing the powder-coated film with toothpaste.

*Red powder coated films:* A very small amount of Red 30 pigment powder (Sensient Co.) is mixed with the black film fragments. The black films stick to the Red 30 pigment powder, changing the color from black to red. The strong dipole moments of both pigments drive the interaction in this case. Baking of the resulting coated films further anneals the added coating. The film fragments are mixed into a clear gel toothpaste and are stable in the gel toothpaste until use. Upon use, the clear gel toothpaste turns blue after about 45 seconds of brushing, when the film fragments disintegrate and release the blue pigment.

In another formulation, red films are made from single layer black films: 5.00g, and 20% glycerin+80% ethanol: 2.50g. The films and the glycerin/ethanol are combined and shaken for 5 min, then Red 30 lake, 29% pure: 0.30g is added. The coated films are then heated at 75°C for 1hr. The resulting films are red.

In another formulation, white films without pigment are treated in the same manner as in the preceding paragraph, and the films are colored red.

## Claims

1. An orally acceptable powder-coated dissolvable single-layer film; wherein the film comprises a polymer matrix and wherein the matrix comprises a pigment that is released upon dissolution of the matrix; wherein the powder coating comprises a pigment, and
wherein the powder coating further comprises a water-insoluble antimicrobial agent.

2. The film according to claim 1 wherein the matrix comprises a hydroxyalkyl cellulose.

3. The film according to claim 2 wherein the hydroxyalkyl cellulose is hydroxypopylmethylcellulose.

4. The film according to any foregoing claim wherein the matrix further comprises a starch.

5. The film according to any foregoing claim wherein the matrix further comprises a plasticizer.

6. The film according to any foregoing claim wherein the matrix comprises propylene glycol.

7. The film according to any foregoing claim wherein the matrix comprises a non-ionic surfactant or emulsifier.

8. The film according to claim 7 wherein the non-ionic surfactant or emulsifier is a polysorbate.

9. The film according to any foregoing claim, wherein the powder coating comprises a pigment comprising particles of titanium dioxide, Ca₂P₂O₇, red iron oxide or D&C Red 30.

10. A method of making a film according to any foregoing claim comprising forming a dissolvable polymer matrix, distributing a powder on the surface of the matrix, and heating the powder-coated film until the powder is adsorbed to the matrix.

## Patentansprüche

1. Oral akzeptabler, pulverbeschichteter, auflösbarer Einzelschichtfilm; wobei der Film eine Polymermatrix umfasst und wobei die Matrix ein Pigment umfasst, das bei Auflösung der Matrix freigesetzt wird; wobei die Pulverbeschichtung ein Pigment umfasst und wobei die Pulverbeschichtung weiterhin ein wasserunlösliches antimikrobiell wirkendes Mittel umfasst.

2. Film nach Anspruch 1, wobei die Matrix eine Hydroxyalkylcellulose umfasst.

3. Film nach Anspruch 2, wobei die Hydroxyalkylcellulose Hydroxypropylmethylcellulose ist.

4. Film nach einem vorhergehenden Anspruch, wobei die Matrix weiterhin eine Stärke umfasst.

5. Film nach einem vorhergehenden Anspruch, wobei die Matrix weiterhin einen Weichmacher umfasst.

6. Film nach einem vorhergehenden Anspruch, wobei die Matrix Propylenglykol umfasst.

7. Film nach einem vorhergehenden Anspruch, wobei die Matrix ein nichtionisches Tensid oder einen nichtionischen Emulgator umfasst.

8. Film nach Anspruch 7, wobei das nichtionische Tensid oder der nichtionische Emulgator ein Polysorbat ist.

9. Film nach einem vorhergehenden Anspruch, wobei die Pulverbeschichtung ein Pigment umfasst, das Teilchen von Titandioxid, Ca₂P₂O₇, Eisenoxidrot oder D&C Red 30 umfasst.

10. Verfahren zur Herstellung eines Films nach einem vorhergehenden Anspruch, das das Bilden einer auflösbaren Polymermatrix, das Verteilen eines Pulvers auf der Oberfläche der Matrix und das Erhitzen des pulverbeschichteten Films, bis das Pulver von der Matrix adsorbiert wurde, umfasst.

## Revendications

1. Un film soluble revêtu de poudre buccalement acceptable ; dans lequel le film comprend une matrice de polymère et dans lequel la matrice comprend un pigment qui est libéré lors de la dissolution de la matrice ; dans lequel le revêtement de poudre comprend un pigment ; et
dans lequel le revêtement de poudre comprend en outre un agent antimicrobien insoluble dans l'eau.

2. Le film selon la revendication 1 dans lequel la matrice comprend une cellulose hydroxyalkyle.

3. Le film selon la revendication 2 dans lequel la cellulose hydroxyalkyle est de la cellulose de méthyle hydropropyle.

4. Le film selon l'une quelconque des revendications précédentes dans lequel la matrice comprend en outre un amidon.

5. Le film selon l'une quelconque des revendications précédentes 1 dans lequel la matrice comprend en outre un plastifiant.

6. Le film selon l'une quelconque des revendications précédentes 1 dans lequel la matrice comprend du propylène glycol.

7. Le film selon l'une quelconque des revendications précédentes 1 dans lequel la matrice comprend un tensioactif non ionique ou un émulsifiant.

8. Le film selon la revendication 7 dans lequel le tensioactif non ionique ou l'émulsifiant est un polysorbate.

9. Le film selon l'une quelconque des revendications précédentes 1, dans lequel le revêtement de poudre est sélectionné parmi le dioxyde de titane, Ca₂P₂O₇, l'oxyde de fer rouge et D&C Red 30.

10. Un procédé de fabrication d'un film selon l'une quelconque des revendications précédentes consistant à former une matrice de polymère soluble, à répartir une poudre sur la surface de la matrice et à chauffer le film revêtu de poudre jusqu'à ce que la poudre soit adsorbée sur la matrice.
